# EUROPEAN PATENT SPECIFICATION

(11) **EP 1 354 224 B1**
(45) Date of publication and mention of the grant of the patent: **27.02.2013**
(21) Application number: 01271968.8
(22) Date of filing: 18.12.2001
(51) Int. Cl.: G01V 15/00, G08B 21/00, G08B 13/22, A61F 13/42, A61F 5/48

(54) **SENSING DEVICE, METHOD OF ITS PRODUCTION, AND USE**
MESSEINRICHTUNG, VERFAHREN ZU IHRER HERSTELLUNG UND BENUTZUNG
DISPOSITIF DE DETECTION ET SON PROCEDE DE PRODUCTION ET SON UTILISATION

(30) Priority: 22.12.2000 DK 200001933
(43) Date of publication of application: 22.10.2003
(73) Proprietor: Bent Thorning Bensen A/S, 3400 Helsingor (DK)
(72) Inventor: BENSEN, Bent, Thorning, DK-3400 Helsingør (DK)
(74) Representative: Schmidt, Johnny Olesen
(86) International application number: PCT/DK2001/000833
(87) International publication number: WO 2002/052302

(56) References cited:
- WO-A1-00/79497
- WO-A1-99/33037
- US-A- 4 578 654
- US-A- 4 583 099
- US-A- 4 694 283
- US-A- 4 792 790
- US-A- 5 291 180

## Description

### 1. BACKGROUND OF THE INVENTION

The present invention relates to a radio frequency resonant circuit sensing device, a method of its production, and use thereof for a fluid sensing device for detecting a fluid in an article or a container; for containing a fluid, in particular a human or animal body fluid; and use thereof for radio frequency resonant circuit tags in detection of theft of articles for sale.

### The Technical Field

Generally, radio frequency resonant circuit devices comprise layered structures of electrical conductors on substrates that comprise inductive and capacitive elements. Electrical insulators separate the electrical conductors that are either electrically connected through separate perforations of the electrical insulators, or electrically connected through folding of continuous electrical conductors that are brought into mutual overlaying relationships.

The resonance frequency of folded resonant circuits depends on the extent of mutual overlaying relationship. This extent of overlay may vary considerably when produced by mass production methods.

On line mass production of folded resonant circuits include rotation impression of coils and capacitor plates of electrical conductors on a continuous moving flexible substrate and subsequent in flight folding of the substrate to obtain a mutual overlay of the capacitor plates. This folding process, however, provides variation in the actual overlay achieved whereby large variations in the resonant parameters of the resonant circuit are obtained.

Variability of the resonance frequency may be important in applications where the radio frequency resonant circuit device is used for sensing the presence of a fluid when high reliability is required e.g. in detecting body fluids from a human or an animal, or in detecting an oil leak from a stationary oil container.

Consequently, there is a need of a radio frequency resonant circuit device that can be mass-produced with a high degree of reproducibility, and a method of producing such a device.

### Prior Art Disclosures

US 4,792,790 discloses a tag-like strip affixable to an article comprising a flexible, non-conductive substrate, at least one inductive element formed from a planar conductive path, a capacitive element formed from superimposed conductive path parts, and a dielectric interposed therebetween, the conductive paths being arranged in at least two surfaces which are superimposed by folding the substrate together forming a closed resonant circuit with the inductive element; said folded substrate avoiding contacts between conductive paths to be pressed through an insulating foil therebetween as in prior art layered structures of resonant circuits.

US 5,291,180 discloses similar LC structures of flexible insulative layers folded back on itself and having on one surface an electrically conductive layer forming multi-turn spirals in a counter turning relationship in a like folded superposition.

US Patent No. 4 646 066 discloses an indicator device and method of measuring incremental environmental exposure of an environmental parameter; said method comprising measuring responses of a target to an electromagnetic interrogation signal; said target including a tuned circuit and an element that is sensitive to environmental exposure, especially exposure to specified fluids e.g. liquids and water vapour, influencing the electronic or ionic conductivity. There is no teaching of parameter influences including the capacitance of the tuned circuit, nor of a measuring method based on changes of transmission of electromagnetic energy of oscillators transmitting electromagnetic energy to the tuned circuits.

WO 99/33037 discloses a sensing device for sensing an external parameter and a method of its production; said sensing device comprising a resonant circuit constituted by a substrate having at least one electrical conductor comprising an inductor and having at least two parts in a mutual overlaying relationship so that said conductor and at least a part of said substrate provide a capacitor; said sensing device being produced by providing said at least one electrical conductor on said substrate, said conductor comprising an inductor and a pair of electrodes, and folding said substrate so that said pair of electrodes are brought into a mutual overlaying relationship; said conductor and substrate being adapted to respond to an external parameter and affect an electric property of said resonant circuit. Nothing is indicated or suggested about providing a shifted resonance frequency of said resonant circuit in order to facilitate mass production with high reproducibility.

US 3 002 260 discloses a method of assembling electric multiple layer coils or windings without causing appreciable bulking of the material and providing an exact registry along a longitudinal axis through the stack formed by the layers thereby facilitating interconnection of terminals. Nothing is indicated or suggested neither about resonant circuits, nor about the terminals of said coils or windings being adapted to form a capacitor.

### 2 DISCLOSURE OF THE INVENTION

### Object of the Invention

It is an object of the present invention to seek to provide an improved radio frequency resonant circuit device.

It is another object to seek to provide such a device that can be mass-produced with high reproducibility of the parameters of the resonant circuit, in particular the capacitance of the capacitor.

It is still another object of the present invention to seek to provide a method and apparatus for reliable detection of a fluid without using any physical connection between a sensor of the fluid and an electronic detection system therefore and without requiring a portable power supply for the fluid sensor.

It is still another object of the present invention to seek to provide a method and apparatus for reliable detection of the level of a fluid in a container, in particular the level of a human body fluid collected in a container; or for detecting the level of an infusion liquid in a container, in particular the level of an infusion liquid in a plastic bag, e.g. of soft plastic that collapses during emptying thereof.

It is still another object of the present invention to provide a method and apparatus for reliable detection of a body fluid from a human or animal body, in particular a leak of body fluid from a human suffering from e.g. urinary and/or faecal incontinence, a human or animal undergoing surgery requiring a drain of body fluid, or a human or animal having e.g. a bleeding wound.

Further objects will be apparent from elsewhere in the description, claims and figures.

### Solution According to the Invention

In an aspect of the present invention there is provided a radio frequency resonant circuit sensing device as claimed in claim 1.

Preferred embodiments are defined in the sub claims 2-11.

A radio frequency resonant circuit sensing device according to the present invention comprising
a folded substrate, said substrate having a back side and a front side and comprises at least one electrical conductor, said electrical conductor being arranged to form an inductor L and at least two electrodes, said conductor and at least two electrodes being positioned on or embedded in said back side, front side, or both, of said substrate;
said substrate being folded along a folding axis F so that said at least two electrodes of said conductor and at least a part of the substrate, or one or more intermediate layers of dielectric media between said at least two electrodes, constitute at least two capacitor plates of a capacitor C in a mutual overlaying relationship;
said inductor and capacitor being electrically connected to form a resonant circuit LC, said resonant circuit having a resonance frequency of radio frequencies depending on the extent of said mutual overlay;
said two capacitor plates being mutually displaced to provide a shifted resonance frequency which differ from the non-shifted resonance frequency at full overlay; and
said shifted resonance frequency depending on the resonance conditions of the resonant circuit.

Generally, the sensing means for sensing a change of resonance conditions of the resonant circuit comprises any of the parameters inductance, capacitance, and resistance, or a combination thereof.

In another aspect of the present invention there is provided a radio frequency resonant circuit sensing as claimed in claim 12.

Preferred embodiments are defined in the sub claims 12-22.

According to the invention there is provided a method of producing a radio frequency resonant circuit sensing device comprising
(a) providing a substrate, said substrate having a back side and a front side and comprises at least one electrical conductor, said electrical conductor being arranged to form an inductor L and at least two electrodes, said conductor and at least two electrodes being positioned on or embedded in said back side, front side, or both, of said substrate;
(b) folding said substrate being folded along a folding axis F so that said at least two electrodes of said conductor and at least a part of the substrate, or one or more intermediate layers of dielectric media between said at least two electrodes, constitute at least two capacitor plates of a capacitor C in a mutual overlaying relationship;
   said inductor and capacitor being electrically connected to form a resonant circuit LC, said resonant circuit having a resonance frequency of radio frequencies depending on the extent of said mutual overlay;
(c) displacing said two capacitor plates in a mutually relationship to provide a shifted resonance frequency which differ from the resonance frequency at full mutual overlay; and
   said shifted resonance frequency depending on the resonance conditions of the resonant circuit;
(d) monitoring said shifted resonance frequency of said resonance circuit in response to exposure to electromagnetic radiation of radio frequencies, and
(e) adjusting said displacement of said two capacitor plates to provide a desired shifted resonance frequency.

According to the present invention, a substrate is folded along a folding axis to provided two capacitor plates, said capacitor plates are mutually displaced to provide a shifted resonance frequency which differ from the non-shifted resonance frequency at full overlay whereby is achieved that an improved radio frequency resonant circuit device is achieved which can be mass produced with high reproducibility of the parameters of the resonant circuit, in particular the capacitance of the capacitor.

Generally, displacing the capacitor plates with respect to each other can vary the extent of mutual overlaying relationship. Thus, a desired shifted resonance frequency can be obtained within a wide range of frequencies ranging from no frequency at all to a non-shifted frequency corresponding to a full overlaying relationship.

A particular advantage is that a target frequency of the resonant circuit can be selected sufficiently different from the non-shifted resonance frequency to allow adjustment of the extent of displacement of the capacitor plates both to increase and/or to reduce the resonant frequency.

This is an advantage compared to prior art folded resonant circuits having full overlaying relationships and operating at non-shifted frequencies. In this case, if at all, the extent of overlaying relation ship can only be reduced, and consequently, the frequency can only be increased.

Normally a decrease in capacity increases the resonance frequency according to ω₀ = (LC)^{-1/2}.

In a preferred embodiment, the displacement of capacitor plates is selected so that a target frequency of shifted resonance frequency is in a range of 30 to 40%, preferably 20 to 30%, most preferably 10 to 20%, particularly 5 to 10% of the non-shifted resonance frequency whereby the influence of variability in a large range of different on-line production conditions is obtained.

In many applications, it is desired that the resonant circuit can be deactivated, e.g. to authorize release of articles of sale, whereby the deactivated resonant circuit is allowed to pass the electromagnetic detection field without triggering an alarm. For this purpose it is known to include a fuse in series in the resonant circuit.

Further, the resonant circuit may comprise a resistor that changes its resistance with changing temperature thereby allowing measurement of e.g. a threshold temperature of a patient.

Accordingly, in a preferred embodiment, the resonant circuit further comprises a resistor, preferably a thermally sensitive resistor or a fuse, respectively.

As it appears, the substrate can be folded so that either of the two surfaces of the substrate face each other, or so that two conductor patterns face each other. In the latter case, it is required that an insulating layer be placed between the conductor path in order to avoid short cuts. This insulating effect can be obtained in any suitable way known to a skilled person.

In a preferred embodiment, said one or more intermediate layers of dielectric media consist of a spacer, preferably a spacer having a variable thickness between said at least two electrodes whereby it is achieved that the spacer both can function as an insulator and a variable capacitor. This is particular advantageous for resonant circuits to function as hinge-type sensing devices sensing relative changes between two elements e.g. in detecting the relative movement of walls of a bag being filled or emptied.

In another preferred embodiment, the spacer is an absorber of fluid whereby a particular sensitive fluid detection can be obtained, e.g. detection of urine.

Optionally, it is preferred that the substrate itself is attractive to the fluid e.g. the substrate is hydrophilic or it comprises a hydrophilic coating.

In a preferred embodiment, the electrical conductor comprises an inductive element having a coil with separated windings, said windings being adapted for receiving a fluid and wholly or partly short cut the circuit whereby a particular sensitive device for sensing fluid can be obtained.

Generally, the electrical conductor can comprises any suitable electrical conducting material that allows the applied conductor pattern to be processed in the on-line production process.

In particular, the conductor part being folded requires a sufficiently flexible material. In some applications flexibility is only required for a part of the conductor pattern.

Suitable materials for conductor patterns are known in the art.

In a preferred embodiment, the electrical conductor comprises a material selected from the group consisting of electrical conducting polymers, paints or, inks; and metallic foils, said material being applied by printing, punching or etching.

For the substrate a similar requirement exists. In particular the region of folding of the substrate must exhibit a suitable flexibility.

Accordingly, in a preferred embodiment, the substrate comprises a flexible material, preferably said flexible material being selected from the group consisting of paper, textile, woven, non-woven, suitable polymer, and plastics.

In order to further improve the ability of the resonant circuit sensing device to sense a fluid, either the inductive element, the capacitive element, the substrate, or a combination of these can be made particular attractive to the fluid, e.g. by coating thereof with a hydrophilic material.

In a preferred embodiment the substrate is attractive to said fluid whereby in an embodiment of the resonant circuit having the induction element positioned on the substrate, the windings of the coil are more likely to get into contact with the fluid.

If the fluid is water or urine, it may short cut the windings and deactivate the resonant circuit.

Accordingly, in a preferred embodiment, the resonant circuit is deactivated by presence of a fluid.

Some applications do not allow the influence of a fluid, e.g. water, which could produce unintended signals, e.g. in theft security systems.

Accordingly, in a preferred embodiment, the substrate is coated with a protective coating, in particular a water repellent layer.

In a particular aspect of the production method according to the invention, there is provided a control of the resonance circuit in flight whereby the control signals can be provided to control units further up the production line.

Accordingly, in a preferred embodiment, the application of said conductor material on said substrate is controlled by monitoring resonance circuit parameters of said folded substrate on line and adjusting said application process conditions to obtain desired parameters for resonance circuit.

In a particular aspect, there is provided an apparatus for detecting a fluid, the apparatus in combination comprising one or more transmitters for transmitting electro-magnetic energy and one or more resonant circuits for receiving electromagnetic energy from said transmitters; wherein said one or more transmitters comprise one or more detectors for detecting changes in one or more characteristics of the one or more transmitters upon changes in characteristics of the one or more resonant circuits by contact thereof with the fluid or contact thereof through an eddy current wherein the one or more resonant circuits are according to the present invention.

In a particularly preferred embodiment, said apparatus comprises one or more transmitters incorporated into a handheld apparatus.

Generally, a resonant circuit sensing device according to the present invention has a number of advantages e.g. that the sensor of the fluid can be separated from the detection electronics for detecting changes in the sensor. This is particularly advantageous when monitoring bodies containing fluid wherein the sensor and the detection electronics used cannot be connected permanently by electrically conducting connectors, or when they can only be connected by such connectors with great difficulties or inconveniences. Also, the oscillators and detection electronics can be simplified.

Various uses of resonant circuits are disclosed in WO 99/33037 the content of which is incorporated herein by reference.

### Uses of the resonant circuit sensing device for detecting a fluid

Accordingly, in still another aspect according to the invention there is provided uses of the method or the apparatus as claimed for detection of fluid level in one or more containers, in particular incontinence containers, specifically diapers; and use for detecting whether a container containing a fluid has been emptied for the fluid in particular emptying of an infusion containing use for monitoring leak of fluid from a container, or a human or animal body, in particular from a human suffering from urinary and/or faecal incontinence; whereby the health care personnel can monitor the hygienic condition of e.g. a diaper. When a body leak has been detected proper care of e.g. changing the diaper can then be taken.

### Sensing devices of a fluid

Still another aspect of the present invention relates to sensors usable for such methods and apparatus in sensing a fluid, more specifically it relates to sensing devices comprising resonant circuits that are responsive to parameters that are able to influence the impedance thereof, e.g. responsive to a fluid.

### Articles comprising the sensing device

In another aspect of the present invention there is provided an article comprising the sensing device.

Preferred articles include, but are not limited to, articles for containing or for taking up fluid or for delivering fluid; such container e.g. being monitored for being filled or being emptied.

In a preferred embodiment, the article consists of a hygienic article for healthy development and maintenance of health.

In a particularly preferred embodiment, the article consists of an absorbent or a bandage in form of a wrap or a trapping used to protect, cover or immobilise an injured or diseased part of a human or an animal, or used during surgery.

In a particularly preferred embodiment, the article consists of an absorbent for urine or faeces, in particular a diaper.

### Use of articles comprising the sensing device

Articles comprising a sensing device according to the present invention are preferably used in a method of monitoring hygienic conditions of one or more patients; said method comprising applying one or more hygienic articles according to the invention to one or more patients, each article having a sensing device with a resonant circuit that differ from each other; and transmitting electromagnetic energy to said resonant circuits.

In an embodiment, the method further comprises monitoring at least one response of said resonant circuits, thereby allowing the prior art techniques of interrogating the resonant circuit to be used.

In a preferred embodiment, the method further comprises monitoring changes of the transmission of electromagnetic energy of one or more oscillators transmitting electromagnetic energy to said resonant circuits, thereby obtaining the advantages of the method according to the present invention.

Further advantages will be apparent from the detailed description.

### Sensing Devices

In its broadest aspect, the sensing device according to the present invention does not necessarily depend on the method of detecting changes of one or more characteristics of the resonant circuit, i.e. for example by detecting the change of the transmission of electromagnetic energy of the oscillators according to the invention, or by detecting a response of the resonant circuit according to prior art methods. However, the specific resonant circuit used typically depends on the parameter to be detected, e.g. a fluid.

Known methods include those referenced in US 4 646 066, e.g. US 4 321 586.

According to the aspect of the invention relating to detecting a fluid, a resonant circuit has one or more characteristics that change by contact between the resonant circuit and a fluid.

In a preferred embodiment of both aspects, the characteristics comprise resistance, capacity, inductance, or any derivative thereof.

By contact of the resonant circuit with the fluid, one or more of the characteristics resistance R, capacitance C and inductance L, or any derivative thereof, e.g. the resonance frequency ω₀ = (LC)^{-½}, or higher harmonics thereof, or the quality factor Q=ωL/R, e.g. at resonance ω=ω₀, change.

When one or more of the characteristics change, the ability of the resonant circuit to receive electromagnetic energy changes. This can be detected by detecting changes in one or more characteristics of one or more oscillators transmitting electromagnetic energy to said resonant circuit.

Generally, sensing devices can be manufactured in any suitable way that allow the external parameters to affect the impedance of the respective resonant circuits.

In the particular aspect of the invention relating to detecting a fluid, resonant circuits can be prepared in any suitable way that ensures the penetration of fluid into the resonant circuit to such an extent that one or more of its characteristics are changed.

In the particular aspect of the method of detecting a fluid, the change of characteristics of the resonant circuit causes a detectable change in one or more of the characteristics of the one or more oscillators.

In a preferred embodiment, the resonant circuit consists of a coil having separated windings that can receive the fluid and short cut the circuit.

The windings can be of any suitable material. In a preferred embodiment, the windings are made of an electrically conducting material selected from the group consisting of metals such as aluminium, copper, tin; an electrically conducting polymer such as polyaniline; and an electrically conducting polymer blend such as poly(p-phenylene vinylene), polyacrylamide, polyaniline and polyethylene, or electrically conducting silicone, or combinations of these.

Specifically useful electric conducting polymers include a flexible, crease resistant, one component, carbon filled ink and coating sold by Emerson & Cuming Speciality Polymers under the trademark Amicon C 932-74. Another useful electric conducting polymer is a highly flexible, crease resistant, one component, silver filled, ink and coating sold by Wacker-Chemie under the trademark Elastosil N 189. Both products are environmentally acceptable.

In the particularly aspect of detecting temperature changes, the resonant circuits can be prepared in any suitable way that ensures the influence of different temperatures to affect the impedance of the resonant circuit to such an extent that one or more of its characteristics are changed.

In the particular aspect of the method of detecting a temperature change, the change of the characteristics of the resonant circuit causes a detectable change in one or more of the characteristics of the one or more oscillators.

The fluid sensing device can be placed in any suitable position to detect the desired fluid. In preferred embodiments, the fluid sensing device is contained in or attached onto a container; or it is embedded in a diaper; or it is embedded in a carrier with an adhesive, such as a sticker, to be attached on a desired location, e.g. onto said container or diaper.

Several resonant circuits can be positioned in one location to encode for given patterns of frequencies, e.g. several small resonant circuits of e.g. different frequencies placed in a diaper can provide a unique identification of e.g. individual patients or elderly being monitored in a hospital or a nursing home.

In a particular aspect the present invention relates to a sensing device for sensing a fluid as claimed in claim 46 that can be mass produced by a printing process or a dispensing process.

A sensing device according to the invention has a number of advantages.

It can be produced by an application process such as a printing process or a dispensing process comprising a series of steps providing a layered structure of the first electrical conducting means, the electrical insulating means, and the second electrical conducting means. Printing or dispensing of suitable electrical conducting materials and electrical insulating materials, respectively, allows very fast and easily controllable production processes to be used.

A particular advantage is obtained, compared to prior art productions techniques of prior art resonant circuits using e.g. copper or tin as the electrical conducting means, in that a flexible resonant circuit can be produced. This is important for many applications wherein the resonant circuit is to be applied on or embedded in a carrier, e.g. a diaper, which can be bent and possible folded.

Further, sensing devices according to the present invention are advantageous in that environmentally acceptable material and/or smaller amounts can be used thereby avoiding discharging used sensing devices including e.g. copper to the environment.

Thus, by selecting a suitable flexible substrate of a material such as paper, textile, woven, non-woven, suitable polymer, plastics, and a flexible electrical conducting polymer for the first and second electrical conducting means, which polymer can be the same or different for the two conducting means, and a suitable flexible insulating material such as polyurethane lacquer for the insulating means, flexible resonant circuits can be provided. A suitable porous material allowing penetration of humidity and/or liquids is Tyvek® supplied by DuPont.

The electrical insulating means is composed of any suitable material for providing electrical insulation. In a preferred embodiment the electrical insulating means consists of polyurethane laquer.

Consequently, in still a further aspect the present invention relates to a method of producing a sensing device.

In a preferred embodiment, the application process involves applying the suitable electrical insulating material onto the first electrical conducting means covering all or parts thereof, e.g. leaving parts for connecting points and areas to be exposed to the fluid during operation of the sensing device uncovered.

In a preferred embodiment, the application process is a printing process such as stencil printing and/or a dispensing process.

The term "dispensing" is intended to mean that a material, here a suitable electrical conducting material or a suitable insulating material which as dispensable, is applied to a substrate material by a suitable application means, including but not limited to application of a continuous fluid of the material, and application of droplets of the material by spraying.

### Transmitter and oscillators

According to the invention an oscillator operates in functional proximity of a resonant circuit typically incorporated in a transmitter of the electromagnetic radiation at radio frequencies.

In the present context the expression "functional proximity of a resonant circuit" is intended to mean that an oscillator in one location radiates electromagnetic energy, e.g. in form of radiowaves or microwaves, which can be fully or partly transferred to a resonant circuit in another location.

Generally, any suitable oscillator can be used, i.e. an oscillator which is able to produce electromagnetic oscillations and to emit electromagnetic energy e.g. in form of radiowaves or microwaves which is fully or partly received by the resonant circuit.

It should be understood that more than one oscillator can operate in functional proximity of said resonant circuits e.g. if more oscillators operates at different frequency ranges, and if changes in different characteristics of the resonant circuit are to be detected.

Generally, an oscillator comprises a generator that generates a high frequency signal of radiant energy e.g. an ac current or voltage, or an impulse, of a frequency around that of the resonant circuit. Generators can be any suitable generator known in the art, e.g. radio frequency signal generators.

In a preferred embodiment, the oscillator comprises a generator generating a signal of electromagnetic energy of a frequency comprising that of the resonant circuit. An oscillator comprises one or more inductors. The inductors can be internal or external to a cabinet housing the oscillator. Preferably there is one or more inductors for each different band of operation for each resonant circuit. Preferably more inductors can be interchanged either manually or automatically. The inductors can be of any suitable form. In a preferred embodiment the inductors are external inductors in form coils.

Functional proximity between the oscillators and the resonant circuits can be achieved in any suitable way. In a preferred embodiment, the inductors are external inductors in form of coils that surrounds the resonant circuit. In a particularly preferred embodiment, the coils are embedded in a bed for monitoring leak of body fluids of a human.

In another preferred embodiment, the oscillators comprise one or more antennas whereby the direction of the electromagnetic radiation, e.g. radio waves, can be more accurately defined. An antenna can be any suitable antenna known in the art, e.g. a dipole. In a preferred embodiment, the antenna comprises a radiator element, transmission lines, and optional transformers, coupled to the inductor, or constituting a part of the inductor.

### Detectors

The apparatus further comprises one or more detectors for detecting one or more changes in one or more characteristics of said oscillators upon changes in the characteristics of one or more resonant circuits.

The characteristics of the oscillators comprise any suitable characteristic.

In a preferred embodiment the characteristics of the oscillator comprise current, voltage, or a derivative thereof such as power, whereby one or more characteristics, or derivatives thereof, of the resonant circuit are detected, e.g. change in frequency, particularly the resonance frequency; change in quality factor; and wholly or partial suppression or restoration of any of these.

In a preferred embodiment one or more of the detectors detect an increase or a decrease of energy loss of one or more of the oscillators.

Further, preferred embodiments of the invention and uses appear from the following detailed description and claims.

### 3. BRIEF DESCRIPTION OF THE DRAWINGS

In the following, the invention is further disclosed with detailed description of preferred embodiments, reference being made to the drawings in which
FIGS. 1A and 1B show a preferred embodiment of a sensing device according to the invention;
FIGS. 2(a)-(g) show an embodiment of the production of an embodiment of the sensing device according to the invention;
FIG. 3 shows an embodiment of one-side application of adhesive onto the substrate for fixation of the folded substrate;
FIG. 4 shows an embodiment of two-sided application of adhesive onto an insert for fixation of the folded substrate;
FIGS. 5A and 5C show a comparison between a fully and a partially exposed coil of a folded substrate;
FIG. 5C shows a double resonant circuit wherein the one circuit has a partially exposed coil, and the other not;
FIG. 6 shows application of a substrate with a protective layer and partially exposed coils;
FIG.7 shows production of a resonant circuit including a resistor;
FIGS. 8A and 8B illustrate production of a diaper;
FIGS. 9, 10, 11A, and 11B show different embodiments of applications of the resonant circuit; and
FIGS. 12A-12D, FIGS. 13A and 13B, and 14 show applications of the resonant circuit inside and outside a collection bag and an infusion bag, respectively, and application to a patient;
FIG. 15 illustrates application of the resonant circuit and a hand held transmitter device for detecting diaper wetness of a child.

### 4. DETAILED DESCRIPTION

### Embodiment of sensing devices according to the invention

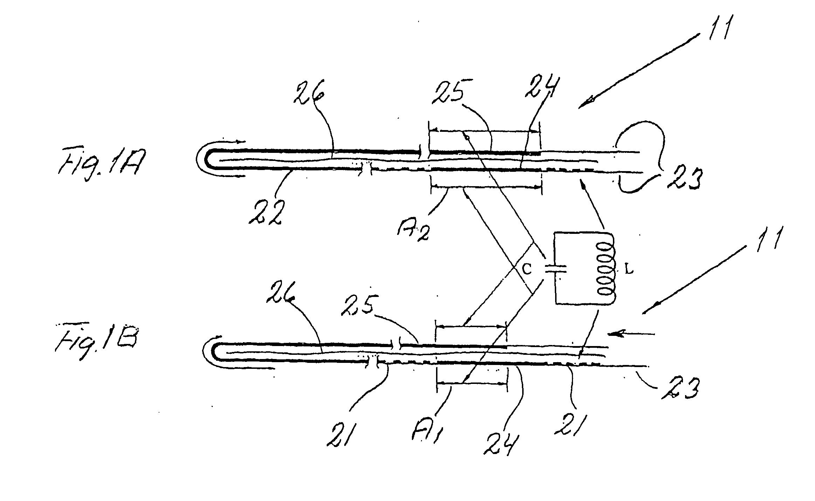

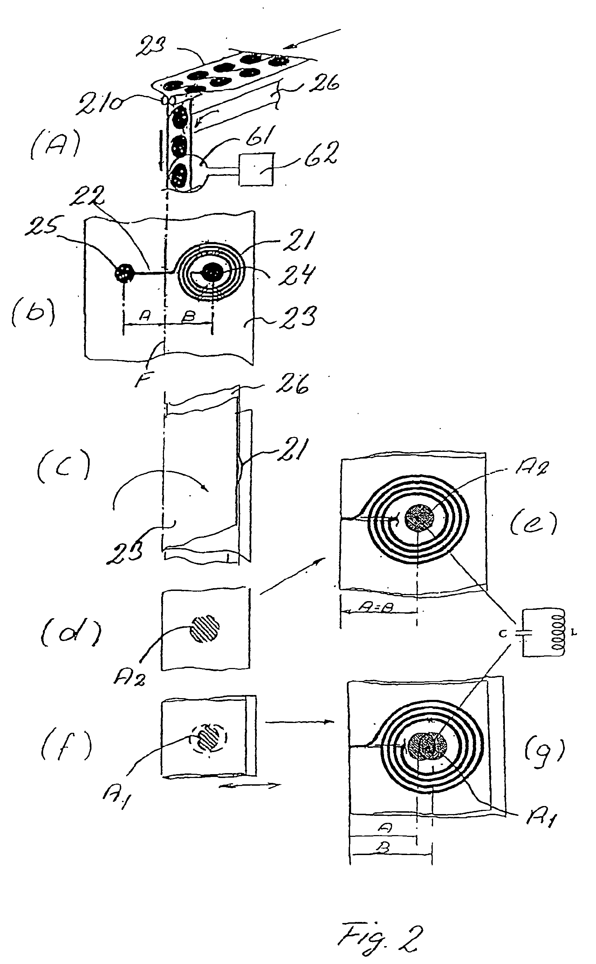

FIGS. 1A and 1B show a preferred embodiment of a sensing device according to the invention; these figures should be read in conjunction with FIG. 2.

A resonant circuit sensing device 11 comprises a substrate, here a flexible material 23 such as paper, textile, plastic, metallic foil, etc. in the form of e.g. a sheet or a foil.

The device further comprises an electrical conductor; here a coil 21 connected to capacitor plates 24,26 with a connecting connector 22 is provided on the substrate 23. As shown in Fig. 2(a), several electrical conductors can be applied to a common substrate band suitable for mass production processes.

At least one electrical conductor comprising an inductor with inductance L here illustrated by windings or a coil 21. Suitable materials for the electrical conductor include a conducting polymer such as an electrical conducting silicone of Wacker Silicones e.g. Semicosil 970 from Wacker-Chemie. Typical forms of the windings are a flat windings but the form is not limited thereto.

### Folding of substrate and Relative Displacement

FIG. 2(c) illustrates folding of the substrate 23 along the folding line F whereby the surfaces of the substrate is brought into an overlaying mutual relation and the connector 22 is folded. A resonant circuit is formed; see the equivalence diagram (LC).

Before the overlaying substrate surfaces are fixed, they can be displaced relatively to each other as indicated by the arrow in FIG. 2(f). Relative displacements produce various extents of mutual overlay in a range from no overlay to full overlay A₂. A smaller extent A₁ of overlay is shown in FIG. 2(f). For more details see FIGS. 2(e) and 2(g) .

Moving either of the two overlaying parts of the substrate can vary the extent of overlay.

Means for displacing the substrate parts are known to the person skilled in the art. They include mechanical manipulators. Preferably, a folding guide 210 is applied to select the position of the folding line F thereby indirectly determining the extent of overlay between the two folded parts of the substrate.

### Insertion of insert

An isolating insert 26 can be inserted between the overlaying parts to prevent short cut when the substrate is folded in the direction bringing the electrical connectors against each other.

The insert can be fixed by any known means, e.g. by applying a hot melt to the two surfaces of the insert, inserting the insert in the hot state, and subsequent cooling once the extent of overlay has been adjusted (see below). Delayed setting adhesives or UV setting adhesives can also be used.

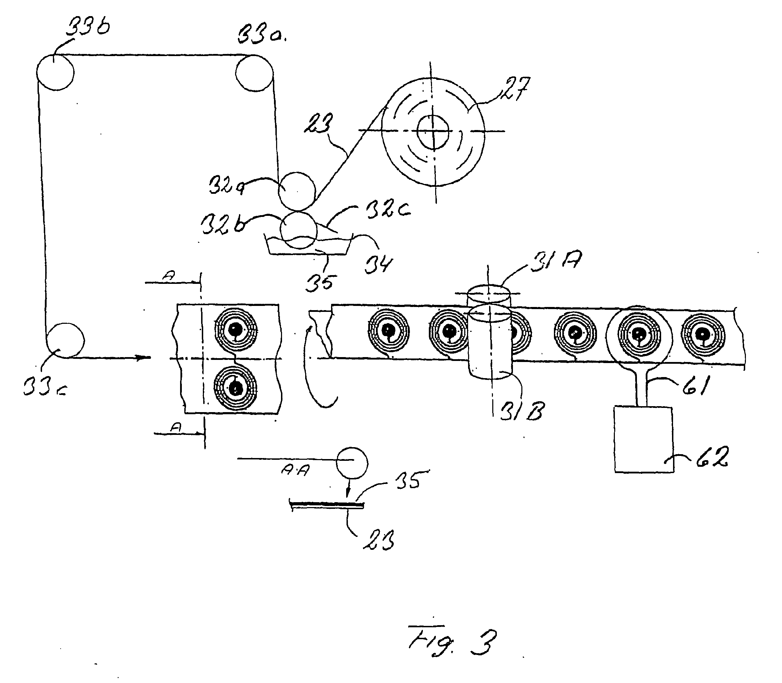

FIG. 3 shows an embodiment of one-side application of adhesive onto the substrate for subsequent fixation of the folded substrate.

The substrate is transferred from a drum 27 to a bath 34 that contains an adhesive 35 here any suitable adhesive known to the skilled person can be used. Doctor blade 32c and rotation drums 32a, 32b provide application of adhesive of the desired thickness to substrate. Although, the substrate is shown as wound up on drum 27, the substrate may be supplied directly from the application unit wherein the conductor is applied to the substrate.

The substrate is folded bringing the adhesive applied surface together. The folded substrate is displacement adjusted (not shown in FIG.3), subsequently compressed between two compression drums 31A and 31B, and monitored by antenna 61 and control system 62 for quality of parameters of the produced resonant circuit.

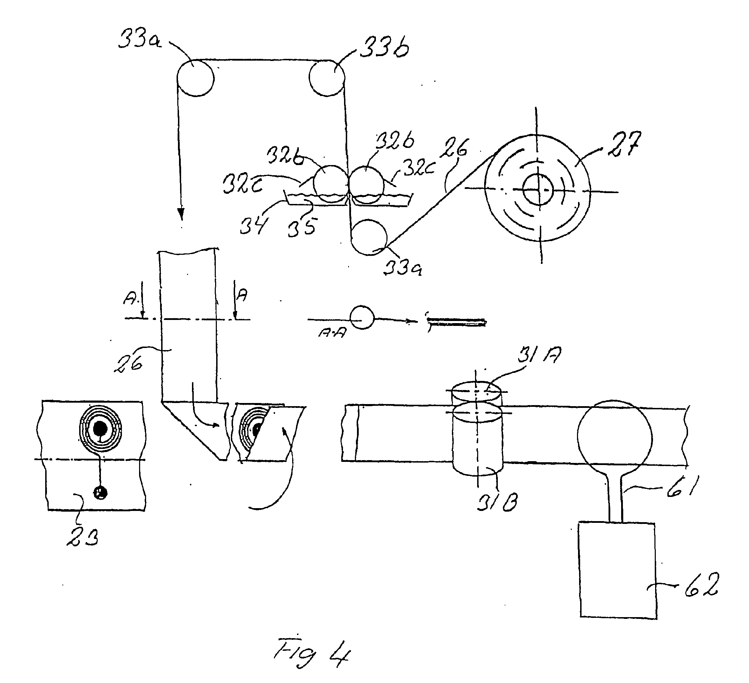

FIG. 4 shows an embodiment of two-sided application of adhesive onto an insert for fixation of the folded substrate.

An adhesive is applied to outer opposite sides of an insert 26 in an application arrangement similar to that shown in FIG.3, except that two application drums 32b and two doctor blades 32c are used.

Instead of direct folding of the substrate as shown in the one-sided application process in FIG.3, the insert is inserted between the folded parts of the substrate.

The subsequent steps are similar to those of FIG.3.

### Quality monitoring

FIG. 2(a) illustrates folding of concurrently moving substrate 23 and insert bands 26 the process of which is monitored on-line by monitoring of the produced resonant circuits. An antenna 61 connected a control unit 62 transmits electromagnetic waves of radio frequencies to the folded substrate. The quality of the produced resonant circuits on the substrate band, e.g. the resonance frequency, is monitored. Comparison with calibration data transmission is made. Control signals for adjustment of process parameters such as speed of substrate and insert bands, folding control means, application process parameters such as e.g. impression pressure, drum speed, etc.

Generally, control test parameters include the frequency of the resonant circuit, but other parameters such as the Q-value or frequency dependent impedance can be contemplated for use in control processes.

### Adhesion to other supports

On a suitable position of the substrate (not shown), the substrate can have an adhesive layer whereby the sensing device can be adhered to e.g. a support surface such as a diaper, a sticker, a label or a tag.

### Partially exposed resonant circuits

As illustrated in FIG. 2(c), a part of the coil may be exposed. This allows a part of the coil to be short cut and provide a shift in frequency.

It should be understood that a resonant circuit of a physical sensing device deviates from the idealized equivalence diagramme of FIGS. 1, 2(e) and (g) mainly due to loss factors such as resistive losses in the leads, energy lost from the circuit by radiation etc. Therefore, including a resistor in the LC equivalence diagram (not shown here) more closely approximates the actual performance of the circuit.

Also, a dedicated resistor may be included in the circuit in order to modify the circuit performance in a particular fashion. The resistor, whether representing loss factors or a combination of loss factors and one or more dedicated resistors, may be coupled in parallel, or in series, or both, with the other components of the resonant circuit.

A person skilled in the art can choose the actual values of the components in the equivalence diagram and optionally a resistor.

It should be noted that the term "being positioned on the surface of said substrate" is intended to include any such positioning wherein the material of the at least one first or second electrical conducting means penetrates wholly or partly into the substrate.

### Production of connector patterns

The method of manufacturing a response circuit provided by the present invention enables an extensive range of options of size and arrangement of devices to be used. Also, a range of printing techniques, punching techniques and etching techniques can be applied, including continuously rotating printing of conductive and dielectric patterns.

Many conductive layers may be printed on top of each other using simple printing techniques without recourse to secondary processes for completing the device.

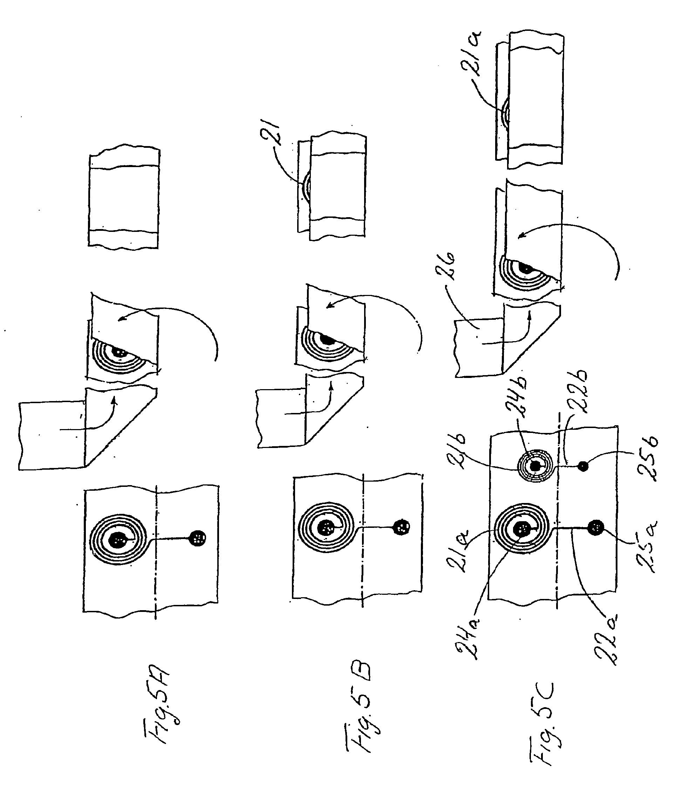

FIGS. 5A and 5C show a comparison between a fully enclosed coil (FIG. 5A) and a partially exposed coil 21 of a folded substrate (FIG. 5B).

FIG. 5C shows a double resonant circuit comprising pairs of resonant circuits wherein one circuit has a partially exposed coil 21a, and the other is fully enclosed.

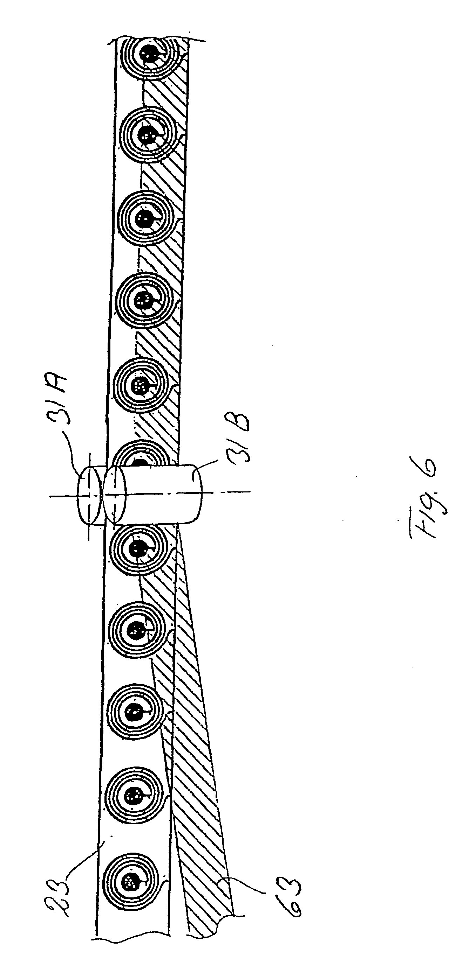

FIG. 6 shows applications of a substrate 23 with a protective layer 63, here a film foil, and partially exposed coils. Application of protective layers can be by any other means e.g. by spraying.

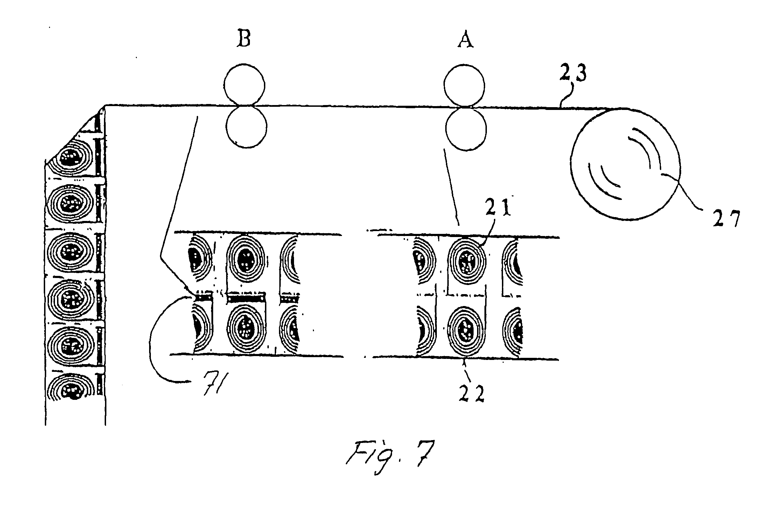

FIG.7 shows production of a resonant circuit including a resistor 71, here the resistor is placed along the folding line in order not to be affected by the subsequent folding process. A substrate 23 is supplied from a drum 27. A rotational impression drum A provides the connector pattern including two coils 21 and 22. Then a rotational impression drum B further down the production line provides the resistor 71. Finally the substrate is folded, e.g. as previously described.

Examples of suitable application apparatus to be used in applying connector patterns to substrates include screen printers and stencil printers from e.g. EKRA, Eduard kraft GmbH Maschinen Fabrik, Bönnigheim, Germany. Specific apparatus can be mentioned e.g. E1^{™} Semi-automative Screen Printer and M2^{™} Semi-automatic Thick-Film Screen Printer, the latter being particularly suited for printing of solder pastes, through-hole coating using vacuum techniques, and multilayer systems. Further suitable apparatus are dispensing apparatus from e.g. Asymtek.

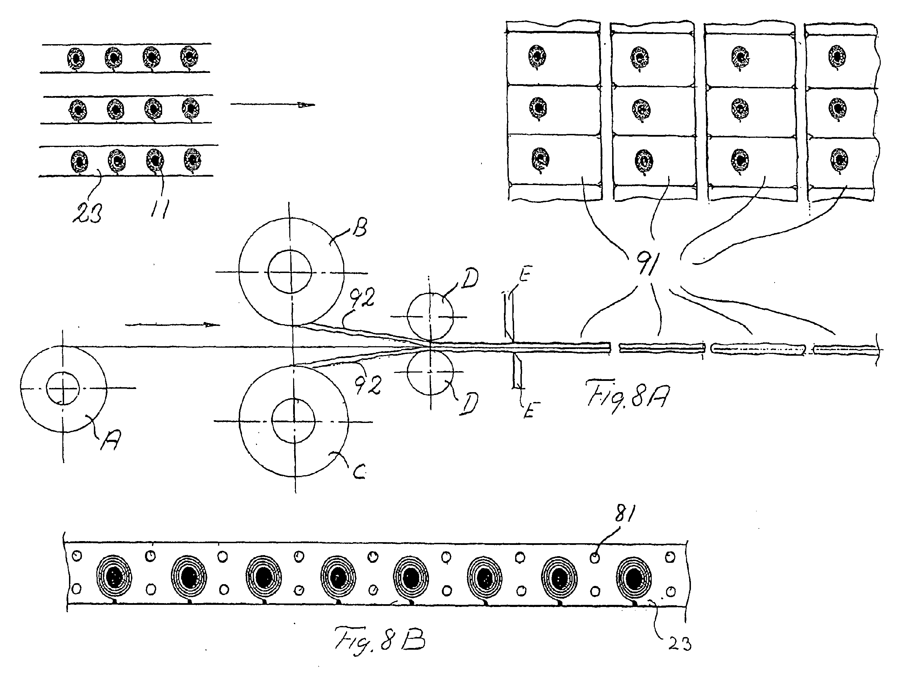

FIGS. 8A and 8B illustrate diaper production wherein several parallel resonant circuits 11 on separate carrier foils, here the carrier foil is constituted by the original substrate 23, are fed from a drum A and laminate on both sides by diaper compatible materials fed from drums B and C and controlled by compression rollers D. The actual diaper produced may include other materials not shown, e.g. water absorbents and water repellent front cover foils. At E, individual sections of each diaper 91 are punched out. It should be noted that the resonant circuits could also be positioned on a common carrier foil.

In another application, the same set up can be used to produce resonant circuit tags for theft security of sales products, in such products the resonant circuit is usually laminated with water repellent foils.

FIG. 8B illustrates perforations 81 in the substrate 23 for controlling the movement thereof.

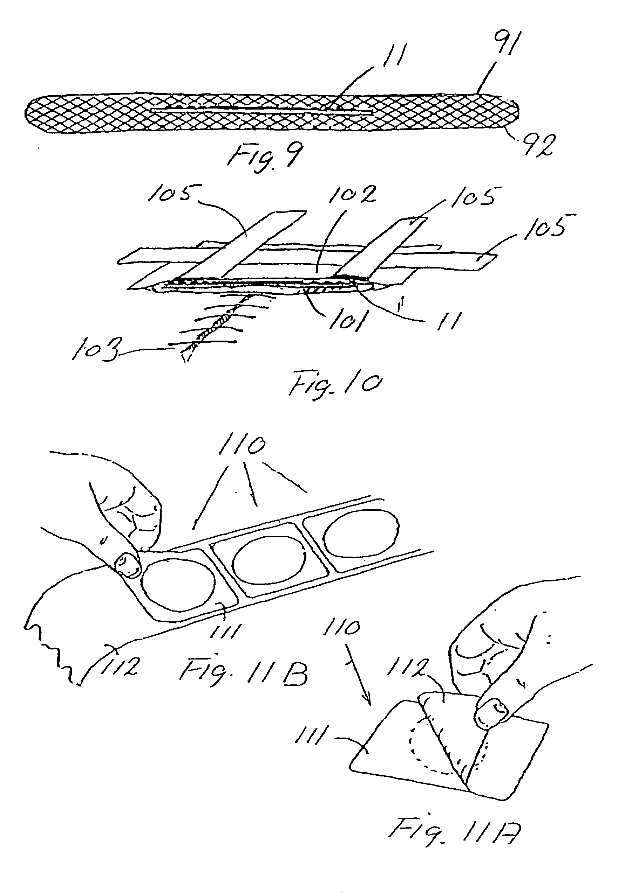

FIGS. 9, 10, and 11A and 11B show different embodiments of applications of the resonant circuit, in particular for a sensing device according to the present invention.

FIG. 9 shows a resonant circuit 11 embedded in a diaper or bandage 91 able to absorb a fluid (not shown), e.g. urine or a body fluid, e.g. blood from a wound, by an absorbing material 92.

FIG. 10 shows a laminated resonant circuit having the resonant circuit 11 embedded between two materials 101, 102 of which one 101 is compatible with human skin and allows humidity and body fluid to penetrate. The laminated circuit can be applied directly on the skin wholly or partly covering a bleeding wound 103. It can be fixed to the skin either by means of a plaster or a tape 105.

FIG. 11A shows an attachable resonant circuit 110 of the type shown in FIG. 10 wherein the resonant circuit is embedded between a carrier material wholly or partly covered with adhesive, e.g. a skin compatible adhesive, material 101 for affixing the resonant circuit to e.g. the skin, or a diaper, and a releasable cover material 102 covering said adhesive properties thereof before use. It should be noted that the resonant circuit can be of the laminated type, or it can be directly adhered to or incorporated in the carrier material.

FIG. 11B shows several attachable resonant circuits 110 similar to that of FIG. 11A provided on an "endless" releasably cover material 101, particularly useful for fast and easy handling and application of many sensing devices, e.g. fluid, to a human body or an article, e.g. a diaper.

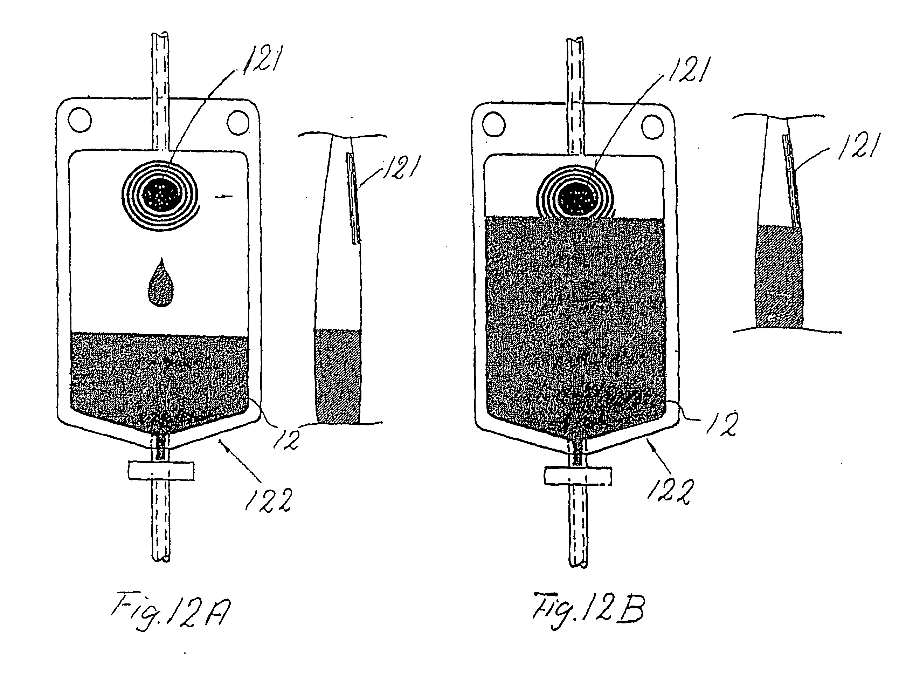

FIGS. 12A and 12B with cross sectional views show application of a fluid sensing device, i.e. a resonant circuit 121 e.g. of the type shown in FIG. 1 or FIG. 2, contained in a container 122, e.g. a collection bag, or drain bag, for monitoring the level of the collected fluid, e.g. drain fluid 12. The characteristics of the resonant circuit are changed upon contact with the fluid, see FIG. 12B.

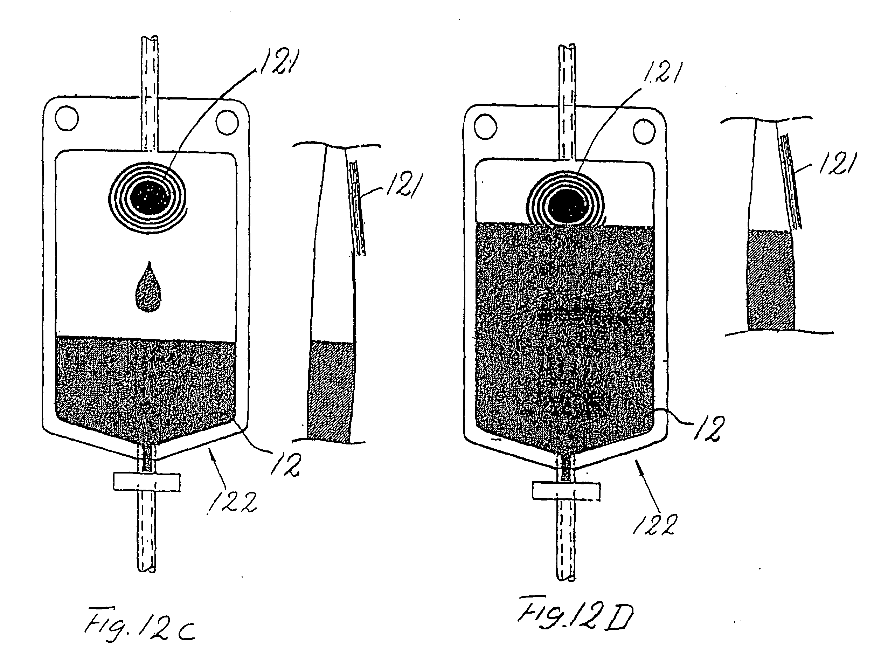

FIGS. 12C and 12D are similar to FIGS. 12A and 12B except that the resonant circuit is placed outside the wall of the collection bag.

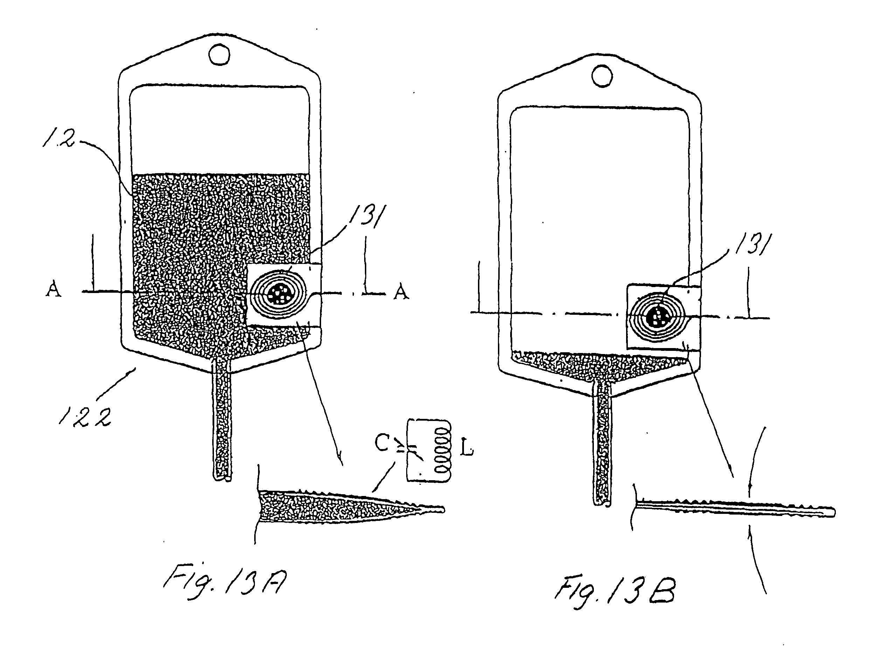

FIGS. 13A and 13B show another application of a resonant circuit 131, e.g. of the type which can be used in combination with a hinge-type resonant circuit in which the folding parts of the substrate are not fixed to each other but the space between them is allowed to vary. The resonant circuit is attached to the outside of a container 122, e.g. an infusion bag, at the fluid level A-A for monitoring the level of a fluid 12 contained therein, when the level of the fluid sinks below the level A-A, the ability of the resonant circuit to receive electromagnetic energy from a transmitter oscillator changes because the capacitor between the serially connected coils changes as the container sides collapse against each other. This embodiment can be used to monitor infusion liquids and indicate an alarm, when the infusion bag is empty.

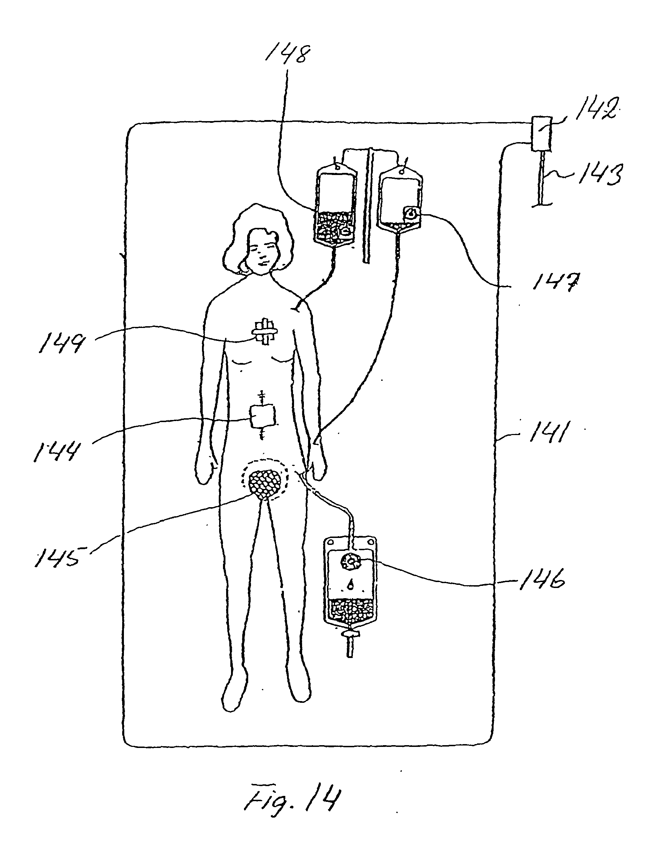

FIG. 14 shows typical applications of the resonant circuit sensing device and apparatus according to the invention.

A human e.g. a patient is positioned within an external inductor 141 of the transmitter 142. The transmitter is powered externally. Electronically communication with external electronics is accomplished through electrical connections 143, e.g. power cables and communications lines.

A resonant circuit sensor 144 e.g. of the type shown in FIG. 9 is show to partly cover a wound which may leak body fluid.

A resonant circuit sensor 145 e.g. of the type shown in FIG. 10 is placed to absorb and monitor a leak of body fluid such as incontinence urine or faeces.

A resonant circuit sensor 146 is applied as a fluid level detector of a body fluid from a drain.

Resonant circuit sensors 147 and 148 are applied as fluid level detectors in infusion containers for monitoring when the containers are empty.

Finally, a resonant circuit sensor 149 e.g. of the type including a resistor in form of a thermistor is applied as a temperature sensor to the body of the patient.

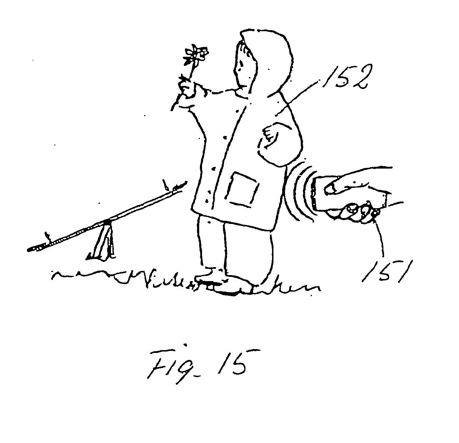

FIG. 15 illustrates application of the resonant circuit in a diaper carried by a child 152 and a hand held transmitter device 151 monitoring wetness of the diaper.

## Claims

1. A sensing device comprising
a substrate (23) having a back side and a front side and at least one electrical conductor (21, 22) arranged to form one inductor (L) and at least two electrodes (24, 25), the one inductor and the at least two electrodes being positioned on or embedded in the back side, front side, or both, of the substrate;
the substrate and the at least one electrical conductor being folded along a folding axis (F) so that the at least two electrodes of the conductor and at least a part of the substrate, or one or more intermediate layers of dielectric media between the at least two electrodes, constitute at least two capacitor plates (24, 25) of a capacitor (C) in a mutual overlaying relationship (A1, A2);
the inductor and capacitor being electrically connected to form a resonant circuit (LC) having a resonance frequency of radio frequencies depending on the extent of the mutual overlay;
the two capacitor plates having a partial overlay to provide a shifted resonance frequency which differs from the non-shifted resonance frequency at full overlay.

2. The device according to claim 1 wherein the shifted resonance frequency is in a range of 30 to 40%, preferably 20 to 30%, most preferably 10 to 20%, particularly 5 to 10% of the non-shifted resonance frequency.

3. The device according to claims 1 or 2 wherein the resonant circuit further comprises a resistor (71), a thermally sensitive resistor, or a fuse.

4. The device according to any of claims 1 to 3 wherein the one or more intermediate layers of dielectric media consist of a spacer (26) between the at least two electrodes, the spacer having variable thickness.

5. The device according to any of claims 1 to 4 wherein the electrical conductor comprises one inductive element having a coil with separated windings adapted for receiving a fluid and wholly or partly short cut the circuit.

6. The device according to any of claims 1 to 5 wherein the electrical conductor comprises a material selected from the group consisting of electrical conducting polymers, paints or inks; and metallic foils, the material being applied by printing, punching or etching.

7. The device according to any of claims 1 to 6 wherein the substrate comprises a flexible material selected from the group consisting of paper, textile, woven, non-woven, suitable polymer, and plastics.

8. The device according to any of claims 1 to 7 wherein the substrate is coated with a protective coating (63).

9. A method of producing a sensing device according to claims 1 to 8, the method comprising
(a) providing a substrate (23) having a back side and a front side and at least one electrical conductor (21,22) arranged to form one inductor (L) and at least two electrodes (24,25), the one inductor and at least two electrodes being positioned on or embedded in the back side, front side, or both, of the substrate;
(b) folding the substrate and the at least one electrical conductor along a folding axis (F) so that the at least two electrodes of the conductor and at least a part of the substrate, or one or more intermediate layers of dielectric media between the at least two electrodes, constitute at least two capacitor plates (24,25) of a capacitor (C) in a mutual overlaying relationship (A1, A2);
the inductor and capacitor being electrically connected to form a resonant circuit (LC) having a resonance frequency of radio frequencies depending on the extent of the mutual overlay;
(c) displacing the two capacitor plates in a mutually relationship to provide a shifted resonance frequency which differ from the resonance frequency at full mutual overlay;
(d) monitoring the shifted resonance frequency of the resonance circuit in response to exposure to electromagnetic radiation of radio frequencies, and
(e) adjusting the displacement of the two capacitor plates to provide a desired shifted resonance frequency.

10. The method according to claim 9 wherein the capacitor is constituted by the at least two electrodes and a spacer o.f variable thickness, the spacer being an absorber of a fluid.

11. The method according to claims 9-10 wherein the substrate is attractive to the fluid.

12. The method according to claims 9-11 wherein the application of conductor material on the substrate is controlled by monitoring resonance circuit parameters of the folded substrate on line and adjusting the application process conditions to obtain desired parameters for the resonance circuit.

13. An apparatus for detecting a fluid, the apparatus in combination comprising one or more transmitters for transmitting electro-magnetic energy and one or more resonant circuits (11) for receiving electromagnetic energy from the transmitters wherein the one or more transmitters comprise one or more detectors for detecting changes in one or more characteristics of the one or more transmitters upon changes in characteristics of the one or more resonant circuits by contact thereof with the fluid or contact thereof through an eddy current wherein the one or more resonant circuits are defined in any of claims 1-8.

14. The apparatus according to claim 13 wherein the one or more transmitters are incorporated into a handheld apparatus.

15. The apparatus according to claim 13 or 14 wherein the one or more resonant circuits are contained in or attached onto one or more containers, embedded in a diaper, or embedded in a carrier with an adhesive such as a sticker.

16. Use of an apparatus according to any of claims 13 to 15 for monitoring a leak from or a supply of fluid to a human or animal body.

17. An article comprising a sensing device as claimed in claims 1 to 8 for containing or for taking up or for delivering of fluid.

18. An article comprising a sensing device as claimed in any of claims 1 to 8 for health development and maintenance of health of a human or an animal.

19. The article according to claim 18, the article consisting of an absorbent or a bandage in form of a wrap or a trapping used to protect, cover or immobilise an injured or diseased part of a human or an animal, or used during surgery, the absorbent being an absorbent for urine or faeces, in particular a diaper.

20. A method of monitoring hygienic conditions of one or more patients, the method comprising applying one or more articles according to any of claims 17 to 19 to one or more patients, each article having a sensing device with a resonant circuit which differs from those of the other articles;
transmitting electromagnetic energy to the resonant circuits;
monitoring at least one response of the resonant circuits; and
monitoring changes of the transmission of electromagnetic energy of one or more transmitters transmitting electromagnetic energy to the resonant circuits.

## Patentansprüche

1. Sensorvorrichtung umfassend
eine gefaltete Substrat (23) mit einer Rückseite und einer Vorderseite und mindestens einem elektrischen Leiter (21,22) angeordnet, um eine Induktivität (L) und mindestens zwei Elektroden (24,25) zu bilden, wobei die eine Induktivität und die mindestens zwei Elektroden auf oder eingebettet in der Rückseite, Vorderseite, oder beides, des Substrats positioniert sind,
wobei das Substrat und die mindestens einem elektrischen Leiter entlang einer Faltachse gefaltet sind (F) so, dass die mindestens zwei Elektroden der Leiter und mindestens ein Teil des Substrats, oder eine oder mehrere Zwischenschichten aus dielektrischen Medien zwischen den mindestens zwei Elektroden, mindestens zwei Kondensatorplatten (24,25) von einem Kondensator (C) in einer gegenseitigen Überlagerung Beziehung (A1, A2) bilden,
wobei die Induktivität und der Kondensator elektrisch verbunden sind, um einen Resonanzkreis (LC) mit einer Resonanzfrequenz von Funkfrequenzen in Abhängigkeit vom Ausmaß der gegenseitigen Überlagerung zu bilden;
wobei die zwei Kondensatorplatten gegeneinander versetzt sind, um einen verschobenen Resonanzfrequenz, die von der nicht-verschobenen Resonanzfrequenz bei vollaufschlagend unterscheiden bereitzustellen.

2. Vorrichtung nach Anspruch 1, wobei die verschobenen Resonanzfrequenz in einem Bereich von 30 bis 40%, vorzugsweise 20 bis 30%, am meisten bevorzugt 10 bis 20%, insbesondere 5 bis 10% der nicht-verschobenen Resonanzfrequenz Anspruch.

3. Vorrichtung nach Ansprüchen 1 oder 2, wobei der Resonanzkreis ferner einen Widerstand (71), einem wärmeempfindlichen Widerstand, oder eine Sicherung umfasst.

4. Vorrichtung nach einem der Ansprüche 1 bis 3, wobei das eine oder mehrere Zwischenschichten aus dielektrischen Medien aus einem Abstandshalter (26) zwischen den mindestens zwei Elektroden bestehen , wobei der Abstandhalter eine variable Dicke aufweist.

5. Vorrichtung nach einem der Ansprüche 1 bis 4, wobei die elektrischen Leiter eine induktive Element eine Spule mit getrennten Wicklungen umfasst, wobei die Wicklungen zum Aufnehmen eines Fluides und ganz oder teilweise Kurzschlusses die Schaltung angepasst ist.

6. Vorrichtung nach einem der Ansprüche 1 bis 5, wobei der elektrische Leiter aus einem Material aus der Gruppe bestehend aus elektrisch leitenden Polymeren, Lacken oder Tinten, und Metallfolien ausgewählt ist, wobei das Material durch Drucken, Stanzen oder Ätzen aufgebracht ist.

7. Vorrichtung nach einem der Ansprüche 1 bis 6, wobei das Substrat ein flexibles Material aufweist, wobei das flexible Material aus der Gruppe bestehend aus Papier, Textil, gewebten, nichtgewebten, geeignetes Polymer und Kunststoffen ausgewählt ist.

8. Vorrichtung nach einem der Ansprüche 1 bis 7, wobei das Substrat mit einer Schutzschicht (63) beschichtet ist.

9. Verfahren zum Erzeugen eines Sensorvorrichtung nach den Ansprüchen 1 bis 8, wobei das Verfahren umfasst
(a) Bereitstellen eines Substrats (23) mit einer Rückseite und einer Vorderseite und mindestens einem elektrischen Leiter (21,22) angeordnet, um eine Induktivität (L) und mindestens zwei Elektroden (24,25) zu bilden, wobei die eine Induktivität und die mindestens zwei Elektroden auf oder eingebettet in der Rückseite, Vorderseite, oder beides, des Substrats positioniert sind,
(b) Falten des Substrates und die mindestens einem elektrischen Leiter entlang einer Faltachse (F), so dass die mindestens zwei Elektroden der Leiter und mindestens ein Teil des Substrats, oder eine oder mehrere Zwischenschichten aus dielektrischen Medien zwischen den mindestens zwei Elektroden, mindestens zwei Kondensatorplatten (24,25) von einem Kondensator (C) in einer gegenseitigen überlappenden Beziehung (A1, A2) bilden,
wobei die Induktivität und der Kondensator elektrisch verbunden sind, um einen Resonanzkreis (LC) mit einer Resonanzfrequenz Funkfrequenzen in Abhängigkeit vom Ausmaß der gegenseitigen Überlagerung zu bilden,
(c) Verschieben der zwei Kondensatorplatten in einer gegenseitig Beziehung zu einer verschobenen Resonanzfrequenz, die von der Resonanzfrequenz abweichen bei voller gegenseitige Überlagerung bereitzustellen,,
(d) Überwachen der verschobenen Resonanzfrequenz des Resonanzkreises in Reaktion auf Bestrahlung mit elektromagnetischer Strahlung von Funkfrequenzen, und
(e) Einstellen der Verschiebung der zwei Kondensatorplatten, um einen gewünschten verschobenen Resonanzfrequenz bereitzustellen.

10. Verfahren nach Anspruch 9, wobei der Kondensator durch die mindestens zwei Elektroden und einem Abstandshalter mit variabler Dicke gebildet wird, wobei der Abstandshalter ein Absorber von Flüssigkeit ist.

11. Verfahren nach einem der Ansprüche 9 bis 10, wobei das Substrat anziehend auf das Fluid.

12. Verfahren nach Ansprüchen 9 bis 11, wobei die Anwendung des leitenden Materials auf dem Substrat durch Überwachung von Parameter des Resonanzkreises des gefalteten Substrates on-line gesteuert wird und Anpassung der Anwendungsprozess Bedingungen um gewünschten Parameter für den Resonanzkreis zu erhalten.

13. Apparat zum Erfassen eines Fluides in Kombination umfassend einem oder mehreren Sendern für elektromagnetische Energie und einen oder mehrere Resonanzkreise (11) zum Empfangen elektromagnetischer Energie von Sendern; wobei ein oder mehrere Sender umfassen, einen oder mehrere Detektoren zum Erfassen von Änderungen einer oder mehrerer Charakteristiken der einen oder mehrere Sender auf Änderungen in Charakteristiken der einen oder mehreren Resonanzkreise durch deren Kontakt mit dem Fluid oder kontaktieren davon durch einen Wirbelstrom, wobei der eine oder mehrere Resonanzkreise sind in einem definierten Ansprüche 1-8.

14. Apparat nach Anspruch 13, wobei die ein oder mehreren Sendern in einem Handhelden Vorrichtung eingebaut sind.

15. Apparat nach Anspruch 13 oder 14, wobei die eine oder mehreren Resonanzkreisen enthalten sind oder befestigt auf einem oder mehreren Behältern, in eine Windel eingebettet sind, oder in einem Träger mit einem Klebstoff, wie einem Aufkleber, eingebettet sind.

16. Verwendung einer Apparat nach einem der Ansprüche 13 bis 15 zur Überwachung eines Leck oder eine Zufuhr von Fluid zu einem menschlichen oder tierischen Körper.

17. Artikel umfassend eine Sensorvorrichtung nach Ansprüche 1 bis 8 zum Enthalten oder zur Aufnahme oder zur Abgabe von Fluid.

18. Artikel umfassend eine Sensorvorrichtung nach einem der Ansprüche 1 bis 8 für gesundheitlichen Entwicklung und Erhaltung der Gesundheit eines Menschen oder eines Tieres.

19. Artikel nach Anspruch 18 bestehend aus einem Absorptionsmittel oder einer Bandage in Form einer Hülle oder einer Trapping zum Schutz, abzudecken oder zu immobilisieren einen verletzten oder kranken Teil eines menschlichen oder tierischen oder während der Operation verwendet, wobei der Absorptionsmittel aus einem Absorptionsmittel für Urin oder Fäkalien, insbesondere einer Windel, ist.

20. Verfahren zum Überwachen hygienischen Bedingungen von einem oder mehreren Patienten, wobei das Verfahren das Aufbringen eines oder mehrerer Artikeln nach einem der Ansprüche 17 bis 19 zu einem oder mehreren Patienten, wobei jeder Artikel mit einer Sensorvorrichtung mit einem Resonanzkreis, die von denen des unterscheidet andere Artikeln, und
Senden elektromagnetischer Energie an die Resonanzkreise,
Überwachen mindestens eines Ansprechens der Resonanzkreise, und
Überwachen von Änderungen der Übertragung von elektromagnetischer Energie von einen oder mehreren Sendern Übertragung elektromagnetischer Energie auf den Resonanzkreisen.

## Revendications

1. Dispositif de détection comprenant
un substrat (23) ayant un côté arrière et un côté avant et au moins un conducteur électrique (21,22) étant agencé pour former une inductance (L) et au moins deux électrodes (24,25), ledit une inductance et au moins deux électrodes étant positionné sur ou incorporé dans ladite face arrière, face avant, ou les deux, dudit substrat,
ledit substrat et le au moins un conducteur électrique étant plié le long d'un axe de pliage (F) de sorte que lesdites au moins deux électrodes dudit conducteur et au moins une partie du substrat, ou une ou plusieurs couches intermédiaires de support diélectrique entre lesdites au moins deux électrodes, constituent au moins deux plaques de condensateur (24,25) d'un capacité (C) dans une relation de superposition mutuelle (A1, A2),
ladite inductance et condensateur étant connecté électriquement pour former un circuit résonnant (LC) ayant une fréquence de résonance de radiofréquences en fonction de la mesure de ladite superposition mutuelle;
lesdites deux plaques de condensateur ayant une superposition partielle pour fournir une décalée fréquence de résonance qui diffère de la non-décalée fréquence résonance à la superposition totale.

2. Dispositif selon la revendication 1, dans lequel à la fréquence de résonance décalée est dans une plage de 30 à 40%, de préférence 20 à 30%, plus préférablement 10 à 20%, en particulier 5 à 10% de la fréquence de résonance non-décalé.

3. Dispositif selon les revendications 1 ou 2, dans lequel ledit circuit résonnant comprend en outre une résistance (71), une résistance sensible à la chaleur, ou d'un fusible.

4. Dispositif selon l'une quelconque des revendications 1 à 3, dans lequel ladite une ou plusieurs couches intermédiaires de milieux diélectriques sont constitués d'un espaceur (26) entre lesdites au moins deux électrodes, lequel ledit espaceur a une épaisseur variable.

5. Dispositif selon l'une quelconque des revendications 1 à 4, dans lequel ledit conducteur électrique comprend un élément inductif comprenant une bobine à enroulements séparés étant adaptée pour recevoir un fluide et tout ou partie de court-circuiter le circuit.

6. Dispositif selon l'une quelconque des revendications 1 à 5, dans lequel ledit conducteur électrique comprend un matériau choisi dans le groupe constitué de polymères conducteurs électriques, des peintures, des encres ou des feuilles métalliques, et, ledit matériau étant appliqué par impression, gravure ou estampage.

7. Dispositif selon l'une quelconque des revendications 1 à 6, dans lequel ledit substrat comprend un matériau flexible choisi dans le groupe constitué par le papier, le textile, tissé, non tissé, polymère approprié, et les matières plastiques.

8. Dispositif selon l'une quelconque des revendications 1 à 7, dans lequel ledit substrat est revêtu d'un revêtement protecteur (63).

9. Procédé de fabrication d'un dispositif de détection selon l'une quelconque des revendications 1 à 8, le procédé comprenant:
(a) fourniture d'un substrat (23) ayant un côté arrière et un côté avant et au moins un conducteur électrique (21,22) étant agencé pour former une inductance (L) et au moins deux électrodes (24,25), ledit une inductance et au moins deux électrodes étant positionné sur ou incorporé dans ladite face arrière, face avant, ou les deux, dudit substrat,
(b) plier ledit substrat et ledit au moins un conducteur électriqu le long d'un axe de pliage (F) de sorte que lesdites au moins deux électrodes dudit conducteur et au moins une partie du substrat, ou une ou plusieurs couches intermédiaires de support diélectrique entre lesdites au moins deux électrodes, à constituer au moins deux plaques de condensateur (24,25) d'un condensateur (C) dans une relation de superposition mutuelle (A1, A2),
ledit inducteur et le condensateur étant relié électriquement pour former un circuit résonnant (LC), ledit circuit résonant ayant une fréquence de résonance de radiofréquences en fonction de l'étendue de ladite superposition mutuelle,
(c) déplacer lesdites deux plaques de condensateur dans une relation mutuellement pour fournir une décalée fréquence de résonance qui diffère de la non- décalée fréquence résonance à la superposition totale,
(d) surveiller ladite décalé fréquence de résonance dudit circuit de résonance en réponse à l'exposition à un rayonnement électromagnétique de radiofréquences, et
(e) régler ledit déplacement desdites deux plaques de condensateur pour fournir une décalée fréquence de résonance souhaitée.

10. Procédé selon la revendication 9, dans lequel ledit condensateur est constitué par ledit au moins deux électrodes et un espaceur d'épaisseur variable, dans lequel ledit espaceur est un absorbeur de fluide.

11. Procédé selon l'une quelconque des revendications 9 à 10, dans lequel ledit substrat est attractif pour ledit fluide.

12. Procédé selon les revendications 9 à 11, dans lequel l'application dudit matériau conducteur sur ledit substrat est contrôlé par les paramètres de surveillance du circuit de résonance dudit substrat replié on-line et ajuste lesdites conditions de procédé applicatif pour obtenir des paramètres souhaités pour le circuit de résonance.

13. Appareil pour détecter un fluide, l'appareil comprenant en combinaison un ou plusieurs émetteurs pour la transmission de l'énergie électromagnétique et un ou plusieurs circuits résonnants (11) pour recevoir l'énergie électromagnétique provenant desdits émetteurs, dans lequel ledit un ou plusieurs émetteurs comprennent un ou plusieurs détecteurs pour détecter des changements dans une ou plusieurs caractéristiques de l'une ou de plusieurs émetteurs sur des changements dans les caractéristiques de l'une ou de plusieurs circuits résonnants par contact de ceux-ci avec le fluide ou communiquer avec celui-ci par un courant de Foucault dans lequel les une ou plusieurs circuits résonnants sont définis dans l'une quelconque des revendications 1 à 8.

14. Appareil selon la revendication 13, dans lequel les une ou plusieurs émetteurs sont incorporés dans un appareil portatif.

15. Appareil selon la revendication 13 ou 14, dans lequel les une ou plusieurs circuits résonnants sont contenus dans ou fixés sur un ou plusieurs conteneurs, incorporés dans une couche-culotte incorporés dans un support avec un adhésif tel qu'un sticker.

16. Utilisation d'un appareil selon l'une quelconque des revendications 13 à 15 pour contrôler une fuite ou à une alimentation de fluide à un corps humain ou animal.

17. Article comprenant un dispositif de détection selon l'une quelconque des revendications 1 à 8 pour contenir ou pour enroler ou pour délivrer de fluide.

18. Article comprenant un dispositif de détection selon l'une quelconque des revendications 1 à 8 pour développement santé et maintenance santé d'un humain ou d'un animal.

19. Article selon la revendication 18, ledit article consistant en un absorbant ou d'un bandage sous la forme d'une pellicule ou d'un piégeage utilisé pour protéger, recouvrir ou immobiliser une partie blessée ou malade d'un homme ou d'un animal, ou utilisé en chirurgie, ledit article consistant en un absorbant pour l'urine ou les matières fécales, en particulier une couche-culotte.

20. Procédé de surveillance des conditions d'hygiène d'un ou plusieurs patients, ledit procédé comprenant l'application d'une ou de plusieurs articles selon l'une quelconque des revendications 17 à 19 à une ou plusieurs patients, chaque article ayant un dispositif de détection avec un circuit de résonance qui diffère de ceux de l' d'autres articles, et
transmission de l'énergie électromagnétique auxdits circuits résonnants,
surveillance d'au moins une réponse des circuits de résonance, and
surveillance des changements de la transmission de l'énergie électromagnétique d'une ou de plusieurs émetteurs d'émission d'énergie électromagnétique à ladite circuits résonnants.
